# EUROPEAN PATENT APPLICATION

(11) **EP 4 286 495 A1**
(43) Date of publication of application: **06.12.2023**
(21) Application number: 22176126.5
(22) Date of filing: 30.05.2022
(51) Int. Cl.: C10G 3/00, C10G 25/00, C10G 25/12, C07C 1/24, C07C 7/13

(54) **SORPTION-ENHANCED CONVERSION OF OXYGENATES TO HYDROCARBONS**

(71) Applicant: Nederlandse Organisatie voor toegepast-natuurwetenschappelijk Onderzoek TNO, 2595 DA 's-Gravenhage (NL)
(72) Inventor: The designation of the inventor has not yet been filed
(74) Representative: Nederlandsch Octrooibureau

(57) **Abstract**

The invention concerns a process for the conversion of oxygenates to hydrocarbons in the presence of a sorbent material capable of adsorbing water. Furthermore the invention concerns a fluidized or fixed bed reactor for conversion of oxygenates to hydrocarbons comprising a catalyst and a sorbent material capable of adsorbing water. The invention also concerns the use of a sorbent material capable of adsorbing water for increasing the conversion of oxygenates to hydrocarbons.

## Description

### Introduction

Interest has risen in renewable sources for producing chemical building blocks: hydrocarbons such as olefins, alkanes and aromatics. By selecting renewable sources instead of petroleum and natural gas the environmental impact of these chemical building blocks reduces significantly. Oxygenates such as methanol and dimethylether (DME) are typical intermediate products obtained in carbon capture and utilisation processes or in bio conversions of organic waste streams. These oxygenates subsequently can be converted into hydrocarbons of interest.

Conversion of oxygenates to hydrocarbons is known in the art. Water is split off from the oxygenate and hydrocarbons deprived of ether and/or alcohol groups are formed. The reaction is exothermic. The specific mix of hydrocarbon formed depends on the reaction conditions such as pressure and temperature and the catalyst selected (Gunawardena et al., Journal of Thermodynamics, vol. 2012, p1-7). There is a vast amount of literature available on selecting reaction conditions and catalyst. The hydrocarbons formed are olefins, alkanes and/or aromatics. The processes typically are carried out at high temperatures to obtain a sufficiently high conversion. Corresponding rapid coking of the catalyst, which typically occurs within 50 - 100 minutes, is mediated by incorporating a continuous decoking step in the process. The optimal temperature depends on the catalyst, targeted hydrocarbons and oxygenate provided. Typically methanol is reacted at temperatures of about 450°C, whilst for example dimethylether is reacted at about 350°C.

Li et al. (ACS Catal. 2021, 11, 7780-7819) provided an overview of methanol towards aromatics conversion (MTA), Tian et al. (ACS Catal. 2015, 5, 1922-1938) and also Topchiev et al. (Catalysts 2019, 9(5), 485) provided an overview of methanol towards olefins conversion (MTO) and Sanz-Martinez et al. (Catalysts 2022, 12, 134) provides state of the art on methanol towards alkanes conversion (MTG). Magomedova et al. (Catalysts, 2019, 9, 485) describes dimethylether to olefins conversion.

In WO 9955650 A1, conversion of methanol to olefins (MTO) is in presence of a catalyst is disclosed. To mediate the rapid coking of the catalyst, the catalyst is regenerated using a gas comprising oxygen at elevated temperatures. Water is removed from the product stream by a quenching step in which the quenching medium is water. US 2019001311 describes MTO process with an acid zeolite catalyst comprising ZSM-5 and bentonite clay. The bentonite clay binds the ZSM-5 particles rendering the catalyst structural integrity. In US2015119617A1 water is removed from the product stream. Removal of water is done by a quenching step of the olefin stream after which the stream is contacted with a molecular sieve comprising zeolite 3A and/or 4A to remove water from the quenched olefin stream. Regeneration of catalyst is performed using a gas comprising oxygen/oxidants at a temperature between 200 and 1500 °C. WO2006/104757 describes a process in which deactivation of the catalyst is reduced. The coked catalyst is regenerated using a regeneration gas wherein the regeneration temperature is not greater than 600 °C. Prior to regenerating the coked catalyst, the regeneration gas can be compressed to condense a portion of the water and may be contacted with a water adsorbent.

### Summary of the invention

The inventors surprisingly found that the conversion of the oxygenate to hydrocarbons process can be increased by introducing a sorbent material capable of adsorbing water. In example 1 surprisingly the conversion of DME in the presence of a sorbent material capable of adsorbing water is much higher than when this sorbent material is water saturated so that the effect of the sorbent material is cancelled. The inventors found that not only the conversion towards hydrocarbons is increased, but also found that the absolute concentration of reactants will be higher which may increase the reaction velocity. Consequently through this adsorption based enhancement a lower reaction temperature can be selected, which is more energy efficient and needs less capital investment for heating equipment and heat resistant material. In addition the operational window for process design for the process of converting oxygenates to hydrocarbons is enlarged; which may open up possibilities for new reaction product mixes. The reaction window is extended to lower temperatures and possibly higher pressure.

In addition the inventors surprisingly found that catalyst deactivation by coking is reduced or even prevented. In example 3 a reactor was operated cyclically, with consecutive steps of oxygenate reaction and sorbent material regeneration. After 50 cycles no catalyst deterioration was observed. Not bound by theory the inventors believe that the lower reaction temperature and the shift in gas composition contribute to this surprising improvement. As the catalyst deactivation is reduced or even prevented, no or less catalyst decoking is needed therewith improving overall process efficiency. A specific catalyst decoking step may be not or less frequent needed.

In a preferred embodiment the process the process comprises two or more reactors comprising the catalyst and the sorbent material capable of adsorbing water. Such combination of reactors can be operated in pressure swing, temperature swing and/or concentration swing modus in which at least one of the reactors is in reaction mode. At the same time one or more of the other reactors is in sorbent regeneration mode. In this way the overall process can be conducted continuously. Such operation has as additional advantage that in case still some catalyst coking occurs, one of the reactors can be in decoking mode. There is no need for a separate decoking reactor and there is more flexibility in reactor design as transport of the catalyst to a specific decoking reactor generally limits the design. During decoking typically also the sorbent is regenerated.

### Detailed Description

The invention relates in a first aspect to a process for the conversion of oxygenates to hydrocarbons comprising:
a) in a reactor contacting a catalyst with the oxygenate in the presence of a sorbent material capable of adsorbing water to form the hydrocarbons; and subsequently
b) regenerating the sorbent material in the same reactor by contacting the sorbent material with a purge gas;
wherein the catalyst and sorbent material are not the same.

Furthermore the invention relates to a fluidized or fixed bed reactor for conversion of oxygenates to hydrocarbons comprising a catalyst for conversion of oxygenates to hydrocarbons and a sorbent material capable of adsorbing water, wherein the catalyst and the sorbent material are not the same.

The invention also concerns the use of a sorbent material capable of adsorbing water for converting oxygenates to hydrocarbons in the presence of a catalyst for conversion of oxygenates to hydrocarbons wherein the catalyst and sorbent material are not the same, preferably wherein the use involves one or more of the following:
- the conversion is increased compared to when the sorbent material is not present,
- the lifetime of the catalyst is elongated compared to when the sorbent material is not present,
- the deactivation of the catalyst is reduced compared to when the sorbent material is not present,
- the reaction rate is increased compared to when the sorbent material is not present, and
- the need for catalyst decoking is reduced preferably prevented compared to when the sorbent material is not present.

The embodiments disclosed in this document apply to all aspects of the invention, i.e. the process for the conversion of oxygenates to hydrocarbons, the fluidized or fixed bed reactor for conversion of oxygenates to hydrocarbons and the use of a sorbent material capable of adsorbing water for the conversion of oxygenates to hydrocarbons in the presence of a catalyst.

### Conversion

The invention relates to the conversion of oxygenates to hydrocarbons. Herein, one or more oxygenate compounds undergo a chemical reaction to form one or more hydrocarbon compounds, Such chemical reactions are known in the art and typically require a catalyst to occur. Based on common knowledge, the skilled person is able to select a combination of oxygenate, catalyst and reaction conditions such as pressure and temperature to steer the reaction towards a desired mix of specific hydrocarbons. In a preferred embodiment at least 50 mole% based on total moles of reaction product excluding water are hydrocarbons, more preferably at least 70 mole%, most preferably at least 90 mole%.

Preferably for the conversion of oxygenates to hydrocarbons the conversion is at least 80%, more preferably at least 90%, even more preferably at least 95%, most preferably at least 98%. The conversion can be calculated by: [ (moles oxygenate fed - moles oxygenate at the end of the reaction) / (moles oxygenate fed) ]; expressed in %. Preferably the reaction product of the conversion of oxygenates to hydrocarbons comprise less than 20 mole% of oxygenates based on total moles of oxygenates fed, more preferably less than 10 mole%, most preferably less than 5 mole%.

In a preferred embodiment at least 50 mole% based on total moles of reaction product excluding water are olefins, more preferably at least 70 mole%, most preferably at least 90 mole%. More preferably at least 50 mole% based on total mole of reaction product excluding water are the sum of ethylene, propylene, 1-butene, 2-butene and isobutylene, more preferably at least 70 mole%, most preferably at least 90 mole%. Even more preferably at least 50 mole% based on total mole of reaction product excluding water are the sum of ethylene and propylene more preferably at least 70 mole%, most preferably at least 90 mole%. For this embodiment preferably the oxygenate is methanol or dimethylether.

In another preferred embodiment at least 50 mole% based on total moles of reaction product excluding water are alkanes, more preferably at least 70 mole%, most preferably at least 90 mole%. More preferably at least 50 mole% based on total mole of reaction product excluding water are the sum of alkanes comprising 2 to 18 carbon atoms, more preferably at least 70 mole%, most preferably at least 90 mole%. Even more preferably at least 50 mole% based on total mole of reaction product excluding water are the sum of ethane, propane, butane, isobutane, pentane, isopentane and neopentane more preferably at least 70 mole%, most preferably at least 90 mole%. For this embodiment preferably the oxygenate is methanol or dimethylether.

In yet another preferred embodiment at least 50 mole% based on total moles of reaction product excluding water are aromatics, more preferably at least 70 mole%, most preferably at least 90 mole%. More preferably at least 50 mole% based on total mole of reaction product excluding water are the sum of benzene, toluene and xylene, more preferably at least 70 mole%, most preferably at least 90 mole%. For this embodiment preferably the oxygenate is methanol or dimethylether.

For all these embodiments the total moles of reaction product excluding water refers to the total moles of reaction product minus the amount of water in the reaction product expressed in mole.

### Oxygenates

Oxygenates are molecules consisting of carbon (C), hydrogen (H) and oxygen (O) atoms For the current invention an oxygenate is typically an ether or an alcohol comprising 1-6 carbon atoms. Preferably the oxygenate consists of hydrogen, carbon and oxygen atoms. Preferably the oxygenate does not comprise a double bond. In a preferred embodiment of the invention the oxygenate is one or more of methanol (MeOH), ethanol (EtOH), isopropyl alcohol (IPA), n-butanol (BuOH), gasoline grade tert-butanol (GTBA), methyl tert-butyl ether (MTBE), tert-amyl methyl ether (TAME), tert-hexyl methyl ether (THEME), ethyl tert-butyl ether (ETBE), tert-amyl ethyl ether (TAEE), dimethylether (DME) or diisopropyl ether (DIPE), more preferably the oxygenate is methanol and/or dimethylether, even more preferably the oxygenate is methanol or even more preferably the oxygenate is dimethylether.

### Hydrocarbons

Hydrocarbons are molecules consisting of carbon (C) and hydrogen (H) atoms. In a preferred embodiment of the invention the hydrocarbons comprise at most 20 carbon atoms, more preferably 2 - 18 carbon atoms. In a preferred embodiment the hydrocarbons are selected from the list consisting of olefins, alkanes and aromatics, more preferably the hydrocarbons are selected from the list consisting of olefins, alkanes and aromatics and the hydrocarbons comprise at most 20 carbon atoms, more preferably 2 - 18 carbon atoms; even more preferably the hydrocarbons are selected from the list consisting of olefins, alkanes and aromatics wherein the olefins comprises 2 to 5 carbon atoms, the alkanes comprises 2 - 18 carbon atoms and the aromatics comprises 6 to 8 carbon atoms.

In a preferred embodiment the hydrocarbons are olefins. In a preferred embodiment the hydrocarbons are alkanes. In a preferred embodiment the hydrocarbons are aromatics.

In a preferred embodiment the oxygenate is methanol and/or dimethylether and the hydrocarbons are olefins. In a preferred embodiment the oxygenate is methanol and/or dimethylether and the hydrocarbons are alkanes. In a preferred embodiment the oxygenate is methanol and/or dimethylether and the hydrocarbons are aromatics.

### Olefins

Olefins are acyclic hydrocarbons with one double bond. Olefins are also named alkenes. In a preferred embodiment of the invention the olefin is one or more of ethylene, propylene, 1-butene, 2-butene, and isobutylene. Olefins are typical building blocks for other chemicals such as polymers.

### Alkanes

Alkanes are acyclic hydrocarbons without a double or triple bond. Alkanes are also named paraffins. Typically alkanes are used as fuels. In a preferred embodiment of the invention the alkanes comprise 2 to 18 carbon atoms, more preferably 3 to 18 carbon atoms. Depending on the application different amounts of carbon atoms are preferred. For synthetic LPG preferably the alkanes comprise 3 to 4 carbon atoms, for naphtha preferably the alkanes comprise 5 to 7 carbon atoms, for petrol preferably the alkanes comprise 4 to 12 carbon atoms, for kerosene preferably the alkanes comprise 11 to 15 carbon atoms, for diesel preferably the alkanes comprise 14 to 18 carbon atoms.

### Aromatics

Aromatics are hydrocarbon compounds comprising an aromatic ring. In a preferred embodiment, the aromatic comprises a phenyl ring. In a preferred embodiment of the invention the aromatic is one or more of benzene, toluene and xylene.

### Catalyst

A vast amount of literature has been published on catalysts for the conversion of oxygenates to hydrocarbons. For example Li et al. (ACS Catal. 2021, 11, 7780-7819) provided an overview of catalysts for conversion towards aromatics (MTA), Tian et al (ACS Catal. 2015, 5, 1922-1938) and also Topchiev et al (Catalysts 2019, 9(5), 485) provided an overview of catalysts for conversion towards olefins (MTO) and Sanz-Martinez et al. (Catalysts 2022, 12, 134) provides state of the art on catalysts for conversion towards alkanes (MTG). Typically catalysts comprising acid-sites are tailored for their purpose.

In a preferred embodiment the catalyst is selected from the list consisting of H-beta, ZSM-5, ZSM-22, MCM-22, mordenite, UZM-9, SAPO (such as SAPO-17,-18, -34, -35, -44, -56) and AIPO, more preferably the catalyst is ZSM-5 or SAPO-34. A catalyst may be fixed to a carrier material and/or bound to a binder material. For the current invention catalyst fixed to a carrier material and/or bound to a binder material is considered as the catalyst.

Although the core structure of some of the catalysts is also known as water adsorbers (for example zeolite), the modifications to make these core structures suitable for catalysis affects the water binding affinity to a great extend in a negative way. Typically the active catalytic sites that are created on the core structure are of acid nature and are created by for example increasing the ratio of silicon (Si) to aluminum (Al) or by introducing phosphorus (P) by contacting with phosphoric acid. These modifications typically obliterate any water adsorbing capacity of the catalyst. Vice versa water adsorbers have no catalytic activity whilst having the same core structure as the catalysts. No catalytic sites are introduced in water adsorbers. Moreover the water adsorption is also influenced by temperature and even though some catalysts may have some remnant water adsorbing capacity at ambient temperature, this capacity is not present at higher temperatures. When catalyst is fixed on a carrier or combined with a binder material and the carrier or binder has some water adsorption capacity this catalyst will not have water adsorption capacity as the carrier or binder will be present in relatively small amounts.

In a preferred embodiment the catalyst has a maximum water adsorption capacity of less than 0.10 gram water per gram catalyst at the temperature and pressure in step a), more preferably less than 0.04 gram water per gram catalyst at the temperature and pressure in step a), even more preferably less than 0.02 gram water per gram catalyst at the temperature and pressure in step a), most preferably less than 0.01 gram water per gram catalyst at the temperature and pressure in step a).

The water adsorption capacity preferably is measured through breakthrough analysis or through thermogravimetric analysis (TGA), preferably through TGA. Both methods are explained in Van Kampen et al. (Adsorption 27, 577-589 (2021)) and Ruthven (Ruthven, D. M. (1984). Principles of adsorption and adsorption processes. John Wiley & Sons.).

In breakthrough analysis, a sample of the catalyst first is dried at 400°C in an atmosphere not comprising water vapour, subsequently at the temperature and pressure in step a) the catalyst is exposed to a gas mixture comprising 40 mole% water and an inert tracer until equilibrated, wherein time integration of the measured outlet composition yields the amount of water adsorbed. The amount of water adsorbed expressed in weight can be divided by the weight of the catalyst sample to deliver the water adsorption capacity in gram water per gram catalyst.

In thermogravimetric analysis a sample of the catalyst first is dried at a temperature of 400°C in an atmosphere not comprising water vapour, subsequently the catalyst is exposed to a gas mixture comprising 40 mole% water for at least 1000 seconds at the temperature and pressure in step a), and the weight increase of the dried catalyst between before and after exposure to the gas mixture comprising 40 mole% water is measured. The water adsorption capacity is the weight increase divided by the weight of the dried catalyst sample.

The form of the catalyst depends on the reactor size and type. In a preferred embodiment the catalyst is a particulate material, preferably the particle size is between 0.10 millimetre and 10 centimetre, more preferably between 0.30 millimetre and 5.0 centimetre, even more preferably between 1.0 centimetre and 5.0 centimetre. Typically the optimal particle size for a fluidized bed reactor is smaller compared to a fixed bed reactor; typically the optimal particle size for an industrial scale reactor is larger than that of a lab reactor. The skilled person is able to select the optimal particle size for a specific reactor design.

### Sorbent material capable of adsorbing water

Sorbent materials capable of adsorbing water are known in the art. Preferably the sorbent material capable of adsorbing water is a molecular sieve, more preferably a zeolite, even more preferably an LTA zeolite. A molecular sieve is a material comprising pores of uniform size. Zeolites are aluminosilicate minerals comprising pores of uniform size. An LTA zeolite is a Linde Type A zeolite. Preferably the sorbent material capable of adsorbing water is a molecular sieve having a pore diameter in the range of 1 - 10 angstrom (A), more preferably in the range of 2-5 A, even more preferably in the range of 3-5 A most preferably in the range of 3-4 A. More preferably the sorbent material capable of adsorbing water is a zeolite having a pore diameter in the range of 1 - 10 A, more preferably in the range of 2-5 A, even more preferably in the range of 3-5 A most preferably in the range of 3-4 A. Even more preferably the sorbent material capable of adsorbing water is an LTA zeolite having a pore diameter in the range of 1 - 10 A, more preferably in the range of 2-5 A, even more preferably in the range of 3-5 A most preferably in the range of 3-4 A.

In a preferred embodiment the sorbent material capable of adsorbing water has a molar ratio of Si to Al ratio between 0.3 : 1.0 and 10.0 : 3.0, more preferably between 0.5:1.0 and 2.0:1.0, most preferably between 0.8:1.0 and 1.2:1.0. An equal amount of Si to Al points to a less acid structure that is well capable of adsorbing water. Preferably the sorbent material capable of adsorbing water has a water adsorption capacity of at least 0.04 gram water per gram sorbent material at the temperature and pressure in step a), more preferably at least 0.06 gram water per gram sorbent material at the temperature and pressure in step a), even more preferably at least 0.08 gram water per gram sorbent material at the temperature and pressure in step a), most preferably at least 0.10 gram water per gram sorbent material at the temperature and pressure in step a).

As for the catalyst, the water adsorption capacity preferably is measured through breakthrough analysis or through thermogravimetric analysis (TGA), preferably through TGA. Both methods are explained in Van Kampen et al. (Adsorption 27, 577-589 (2021)) and Ruthven (Ruthven, D. M. (1984). Principles of adsorption and adsorption processes. John Wiley & Sons.).

In breakthrough analysis, a sample of the sorbent material capable of adsorbing water first is dried at 400°C in an atmosphere not comprising water vapour, subsequently at the temperature and pressure in step a) the sorbent material capable of adsorbing water is exposed to a gas mixture comprising 40 mole% water and an inert tracer until equilibrated, wherein time integration of the measured outlet composition yields the amount of water adsorbed. The amount of water adsorbed expressed in weight can be divided by the weight of the sorbent material capable of adsorbing water sample to deliver the water adsorption capacity in gram water per gram sorbent material capable of adsorbing water.

In thermogravimetric analysis a sample of the sorbent material capable of adsorbing water first is dried at a temperature of 400°C in an atmosphere not comprising water vapour, subsequently the sorbent material capable of adsorbing water is exposed to a gas mixture comprising 40 mole% water for at least 1000 seconds at the temperature and pressure in step a), and the weight increase of the dried sorbent material capable of adsorbing water between before and after exposure to the gas mixture comprising 40 mole% water is measured. The water adsorption capacity is the weight increase divided by the original weight of the dried sorbent material capable of adsorbing water sample.

In a preferred embodiment the catalyst and sorbent material capable of adsorbing water are not bound to each other. Binding may decrease the catalytic activity of the catalyst and/or the water adsorption capacity of the sorbent material. Moreover binding is expensive and provides for less freedom in selecting the ratio of catalyst and sorbent material capable of adsorbing water.

In a preferred embodiment the weight ratio of catalyst to sorbent material capable of adsorbing water is between 1.0:10.0 and 10.0:1.0, more preferably between 1.0:10.0 and 5.0:1.0, even more preferably between 1.0:10.0 and 2.0:1.0, most preferably between 1.0:5.0 and 1.0:1.0. Within these ratios good water adsorption capacity is obtained at sufficient reaction rate.

The form of the sorbent material capable of adsorbing water depends on the reactor size and type. In a preferred embodiment the sorbent material capable of adsorbing water is a particulate material, preferably the particle size is between 0.10 millimetre and 10 centimetre, more preferably between 0.30 millimetre and 5.0 centimetre, even more preferably between 1.0 centimetre and 5.0 centimetre. Typically the optimal particle size for a fluidized bed reactor is smaller compared to a fixed bed reactor; typically the optimal particle size for an industrial scale reactor is larger than that of a lab reactor. The skilled person is able to select the optimal particle size for a specific reactor design.

In a preferred embodiment at the start of step a) the sorbent material capable of adsorbing water comprises less than 0.10 gram water per gram sorbent material at the temperature and pressure in step a), even more preferably less than 0.04 gram water per gram sorbent material at the temperature and pressure in step a), even more preferably less than 0.02 gram water per gram sorbent material at the temperature and pressure in step a), most preferably less than 0.01 gram water per gram sorbent material at the temperature and pressure in step a).

### Purge gas

In a preferred embodiment the purge gas is air, nitrogen, hydrogen or hydrocarbon gas, more preferably air. Preferably the purge gas comprises less than 5.0 mole% water on total of the purge gas, preferably less than 1.0 mole% water, most preferably less than 0.5 mole% water.

### Process

An aspect of the invention concerns a process for the conversion of oxygenates to hydrocarbons. The process comprises a reaction step a) in which the reaction product is formed and a regeneration step b) in which the sorbent material capable of adsorbing water is regenerated. Regeneration of the sorbent material capable of adsorbing water concerns desorbing of water from the sorbent material. In this way the sorbent material is ready for adsorbing water in a next reaction step a). During the reaction step a) next to the formation of the reaction product also water is adsorbed by the sorbent material capable of adsorbing water.

The invention concerns contacting in a reactor. In a preferred embodiment contacting is by providing a feed, more preferably by providing a continuous feed. In a preferred embodiment contacting is by providing a feed and the feed is a feed gas.

In a preferred embodiment the temperature in step a) is between 200°C and 500°C, more preferably between 200°C and 400°C, even more preferably between 200°C and 350°C, most preferably between 225°C and 275°C. This temperature is lower than that of conventional processes. The inventors surprisingly found that even at these lower temperatures excellent conversion could be achieved. Moreover the lower temperature provides for slower or even no coke formation. Typically the catalyst deactivates due to coke formation. In a preferred embodiment the oxygenate is DME and the temperature in step a) is between 200°C and 500°C, more preferably between 200°C and 400°C, even more preferably between 200°C and 350°C, most preferably between 225°C and 275°C or the oxygenate is methanol and the temperature in step a) is between 200°C and 500°C, more preferably between 275°C and 400°C, even more preferably between 300°C and 400°C, most preferably between 300°C and 350°C.

In a preferred embodiment the pressure in step a) is between 1.0 and 30 bara, more preferably between 1.0 and 20 bara, even more preferably between 2.0 and 10 bara, most preferably between 4.0 and 8.0 bara. The selection of the pressure in step a) is related to several aspects. A higher pressure in step a) enables a larger pressure difference between step a) and step b), which is beneficial for water desorption in step b) in case pressure is selected as the driving force for water desorption. Such pressure swing operation is in general an efficient way to operate the process according to the invention, as a pressure drop can be effectuated within a short timespan and uses relatively little energy. Due to that water is removed during the reaction step, which is not the case for conventional processes, increasing the pressure tends to affect the conversion to a lesser extend. In some cases it has been reported that pressure influences the selectivity. In the art processes for the conversion of oxygenates to hydrocarbons typically operate at ambient pressure, but also operational pressures up to 30 bara have been reported.

The conditions in the reactor between step a) and step b) differ in temperature, pressure and/or feed gas composition. This is typical for a swing process. The water holding capacity of the sorbent depends on temperature and pressure. During regeneration step b) the reactor undergoes a change in temperature, pressure and/or feed gas composition to desorb the water from the sorbent material efficiently. The purge gas in step b) carries the water away. In a preferred embodiment the temperature in step b) is at least 100°C higher than the temperature of step a) and/or the pressure of step b) is at least 0.9 bar lower than the pressure of step a), more preferably the temperature in step b) is at least 150°C higher than the temperature of step a) and/or the pressure of step b) is at least 3.0 bar lower than the pressure of step a), even more preferably the temperature in step b) is at least 200°C higher than the temperature of step a) and/or the pressure of step b) is at least 5.0 bar lower than the pressure of step a). In a preferred embodiment the temperature in step b) is at least 100°C higher than the temperature of step a), more preferably the temperature in step b) is at least 150°C higher than the temperature of step a), even more preferably the temperature in step b) is at least 200°C higher than the temperature of step a). In a preferred embodiment the pressure of step b) is at least 0.9 bar lower than the pressure of step a), more preferably the pressure of step b) is at least 3.0 bar lower than the pressure of step a), even more preferably the pressure of step b) is at least 5.0 bar lower than the pressure of step a).

In a preferred embodiment the temperature in step b) is between 200°C and 500°C, more preferably between 200°C and 400°C, even more preferably between 200°C and 350°C, most preferably between 225°C and 275°C. In case the process comprises a temperature swing, preferably the temperature in step b) is between 300°C and 700°C, more preferably between 400°C and 600°C, even more preferably between 400°C and 500°C. In a preferred embodiment the pressure in step b) is between 1.0 and 30 bara, more preferably between 1.0 and 20 bara, even more preferably between 2.0 and 10 bara, most preferably between 4.0 and 8.0 bara. In case the process comprises a pressure swing, preferably the pressure in step b) is between 2.0 and 20 bara, more preferably between 1.0 and 10 bara, even more preferably between 0.5 and 5 bara, most preferably between 0.1 and 2 bara.

In a preferred embodiment the process is operated cyclically wherein step b) is followed by another occurrence of step a). In this way a reactor of the process alternates between mode a) and mode b), optionally in combination with additional modes. Considering for a manufacturing plant a continuous process is preferred the process preferably comprise two or more reactors, more preferably three or more reactors comprising the catalyst and the sorbent material capable of adsorbing water. For this embodiment preferably at least one reactor of the two or three or more reactors is in mode a). By having two or more reactors, one reactor can be in reaction mode a) whilst the other reactor is in regeneration mode b). The moment the sorbent material capable of adsorbing water in the reactor that is in reaction mode a) is saturated with water the feed flow can be switched to the reactor that was in regeneration mode b) and the reaction can continue in this reactor; so this reactor goes into reaction mode a). Meanwhile the reactor comprising the water saturated sorbent material can go into regeneration mode b). In this way a continuous feed flow and product flow can be maintained. In some situations mode a) and mode b) are not of equal length. Therefore in a preferred embodiment the process comprises a step c) idle mode. During idle mode no gas is fed to the reactor.

The inventors found that for the inventive process less or no coking of the catalyst tends to occur. This may reduce or even eliminate the need for decoking of the catalyst. Not bound by theory the inventors believe that the lower temperature and/or the removal of water during the reaction and/or the intermitted sorbent regeneration have a positive effect on coke formation. Still there may be situations in which the catalyst needs decoking. During catalyst decoking coke is removed by contacting the catalyst with an oxygen comprising gas at a temperature above 400°C, preferably above 500°C, more preferably above 600°C. In a preferred embodiment the process of the invention comprises a step d) decoking the catalyst by contacting with an oxygen containing gas at a temperature above 400°C, preferably above 500°C, more preferably above 600°C. In a preferred embodiment the catalyst is de-coked in the reactor comprising the catalyst and sorbent material capable of adsorbing water. In this way catalyst decoking and sorbent regeneration is simultaneous. For this embodiment preferably the reactor is a fixed bed reactor. Preferably the process is operated cyclically wherein step b) is followed by step a) and step d) is performed at most once every 3 cycles, more preferably at most once every 6 cycles, most preferably at most once every 9 cycles.

In an alternative preferred embodiment the catalyst is de-coked in a separate decoking reactor. For this embodiment preferably the reactor is a fluidized bed reactor.

### Reactor

The invention furthermore concerns a fluidized or fixed bed reactor for conversion of oxygenates to hydrocarbons comprising a catalyst for conversion of oxygenates to hydrocarbons and a sorbent material capable of adsorbing water, wherein the catalyst and the sorbent material are not the same. In a preferred embodiment the invention concerns 2 or more of these reactors. Preferably the reactor is a fixed bed reactor.

In a preferred embodiment the catalyst is selected from the list consisting of H-beta, ZSM-5, ZSM-22, MCM-22, mordenite, UZM-9, SAPO (such as SAPO-17,-18, -34, -35, -44, -56) and AIPO, preferably the catalyst is ZSM-5 or SAPO-34. In a preferred embodiment the sorbent material capable of adsorbing water is a molecular sieve having a pore diameter in the range of 2-5 A, more preferably a zeolite having a pore diameter in the range of 2-5 A, even more preferably an LTA zeolite having a pore diameter in the range of 2-5 A.

In a preferred embodiment the weight ratio of catalyst to sorbent material capable of adsorbing water is between 1.0:10.0 and 10:1.0, preferably between 1.0:10.0 and 5.0:1.0. In a preferred embodiment the sorbent material capable of adsorbing water has a water adsorption capacity of at least 0.04 gram water per gram sorbent material at the temperature and pressure in step a), preferably at least 0.06 gram water per gram sorbent material at the temperature and pressure in step a), more preferably at least 0.08 gram water per gram sorbent material at the temperature and pressure in step a), most preferably at least 0.10 gram water per gram sorbent material at the temperature and pressure in step a).

In a preferred embodiment the catalyst has a water adsorption capacity of less than 0.10 gram water per gram catalyst at the temperature and pressure in step a), preferably less than 0.04 gram water per gram catalyst at the temperature and pressure in step a). In a preferred embodiment the catalyst and sorbent material capable of adsorbing water are not bound to each other.

In a preferred embodiment the oxygenate is one or more of methanol (MeOH), ethanol (EtOH), isopropyl alcohol (IPA), n-butanol (BuOH), gasoline grade tert-butanol (GTBA), methyl tert-butyl ether (MTBE), tert-amyl methyl ether (TAME), tert-hexyl methyl ether (THEME), ethyl tert-butyl ether (ETBE), tert-amyl ethyl ether (TAEE), dimethylether (DME) or diisopropyl ether (DIPE), preferably the oxygenate is methanol and/or dimethylether, more preferably the oxygenate is methanol. In a preferred embodiment the hydrocarbon is selected from the group consisting of olefins, alkanes, aromatics and any combination thereof, wherein preferably the olefin is one or more of ethylene, propylene, 1-butene, 2-butene, and isobutylene, the alkane is one or more of alkanes comprising 2 to 18 carbon atoms and the aromatic is one or more of benzene, toluene and xylene.

### Use

The invention also concerns the use of a sorbent material capable of adsorbing water for converting oxygenates to hydrocarbons in the presence of a catalyst for conversion of oxygenates to hydrocarbons wherein the catalyst and sorbent material are not the same, preferably wherein the use involves one or more of the following:
- the conversion is increased compared to when the sorbent material is not present,
- the lifetime of the catalyst is elongated compared to when the sorbent material is not present,
- the deactivation of the catalyst is reduced compared to when the sorbent material is not present,
- the reaction rate is increased compared to when the sorbent material is not present, and
- the need for catalyst decoking is reduced preferably prevented compared to when the sorbent material is not present.

Preferably, the increase, the elongation and/or the reduction is compared to when the sorbent material is not present and the same amount of catalyst is present.

So in one embodiment the invention concerns the use of a sorbent material capable of adsorbing water for increasing the conversion of oxygenates to hydrocarbons in the presence of a catalyst for conversion of oxygenates to hydrocarbons wherein the catalyst and sorbent material are not the same and the increase is compared to when the sorbent material is not present.

In another embodiment the invention concerns the use of a sorbent material capable of adsorbing water for increasing the lifetime of the catalyst in a process for converting oxygenates to hydrocarbons in the presence of a catalyst for conversion of oxygenates to hydrocarbons wherein the catalyst and sorbent material are not the same and the increase is compared to when the sorbent material is not present.

In another embodiment the invention concerns the use of a sorbent material capable of adsorbing water for reduction of deactivation of the catalyst in a process for converting oxygenates to hydrocarbons in the presence of a catalyst for conversion of oxygenates to hydrocarbons wherein the catalyst and sorbent material are not the same and the reduction is compared to when the sorbent material is not present.

In another embodiment the invention concerns the use of a sorbent material capable of adsorbing water for increasing the reaction rate in a process for converting oxygenates to hydrocarbons in the presence of a catalyst for conversion of oxygenates to hydrocarbons wherein the catalyst and sorbent material are not the same and the increase is compared to when the sorbent material is not present.

In another embodiment the invention concerns the use of a sorbent material capable of adsorbing water for reduction of the need of catalyst decoking in a process for converting oxygenates to hydrocarbons in the presence of a catalyst for conversion of oxygenates to hydrocarbons wherein the catalyst and sorbent material are not the same and the reduction is compared to when the sorbent material is not present.

In a preferred embodiment the catalyst is selected from the list consisting of H-beta, ZSM-5, ZSM-22, MCM-22, mordenite, UZM-9, SAPO (such as SAPO-17,-18, -34, -35, -44, -56) and AIPO, preferably the catalyst is ZSM-5 or SAPO-34. In a preferred embodiment the sorbent material capable of adsorbing water is a molecular sieve having a pore diameter in the range of 2-5 A, more preferably a zeolite having a pore diameter in the range of 2-5 A, even more preferably an LTA zeolite having a pore diameter in the range of 2-5 A.

In a preferred embodiment the weight ratio of catalyst to sorbent material capable of adsorbing water is between 1.0:10.0 and 10:1.0, preferably between 1.0:10.0 and 5.0:1.0. In a preferred embodiment the sorbent material capable of adsorbing water has a water adsorption capacity of at least 0.04 gram water per gram sorbent material at the temperature and pressure in step a), preferably at least 0.06 gram water per gram sorbent material at the temperature and pressure in step a), more preferably at least 0.08 gram water per gram sorbent material at the temperature and pressure in step a), most preferably at least 0.10 gram water per gram sorbent material at the temperature and pressure in step a).

In a preferred embodiment the catalyst has a water adsorption capacity of less than 0.10 gram water per gram catalyst at the temperature and pressure in step a), preferably less than 0.04 gram water per gram catalyst at the temperature and pressure in step a). In a preferred embodiment the catalyst and sorbent material capable of adsorbing water are not bound to each other.

In a preferred embodiment the oxygenate is one or more of methanol (MeOH), ethanol (EtOH), isopropyl alcohol (IPA), n-butanol (BuOH), gasoline grade tert-butanol (GTBA), methyl tert-butyl ether (MTBE), tert-amyl methyl ether (TAME), tert-hexyl methyl ether (THEME), ethyl tert-butyl ether (ETBE), tert-amyl ethyl ether (TAEE), dimethylether (DME) or diisopropyl ether (DIPE), preferably the oxygenate is methanol and/or dimethylether, more preferably the oxygenate is methanol. In a preferred embodiment the hydrocarbon is selected from the group consisting of olefins, alkanes, aromatics and any combination thereof, wherein preferably the olefin is one or more of ethylene, propylene, 1-butene, 2-butene, and isobutylene, the alkane is one or more of alkanes comprising 2 to 18 carbon atoms and the aromatic is one or more of benzene, toluene and xylene.

In a preferred embodiment the use is in a fluidized bed reactor or a fixed bed reactor, preferably in a fixed bed reactor. In a preferred embodiment the use is at a temperature between 200°C and 500°C, preferably between 200°C and 400°C, even more preferably between 200°C and 350°C, most preferably between 225°C and 275°C. In a preferred embodiment the use is at a pressure between 1.0 and 30 bara, preferably between 1.0 and 20 bara.

### General Definitions

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (e.g. about 10) preferably means that the value may be the given value more or less 1% of the value.

The present invention has been described above with reference to a number of exemplary embodiments. Modifications and alternative implementations of some parts or elements are possible, and are included in the scope of protection as defined in the appended claims. All citations of literature and patent documents are hereby incorporated by reference.

Bara stands for bar absolute and concerns the pressure that is zero-referenced against a perfect vacuum. 1 bar is 100,000 N/m². 1 bar is 100,000 Pascal.

### Description of the figures

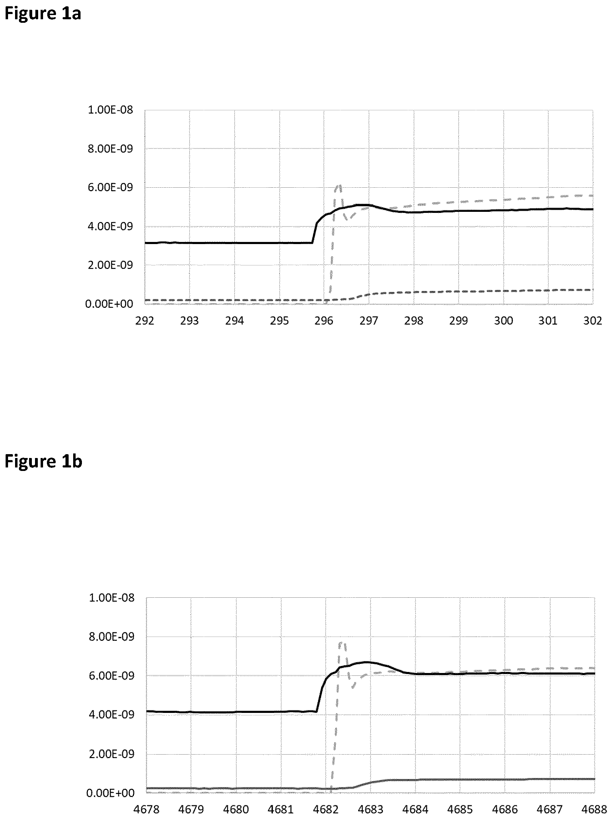

Figure 1a depicts the output concentration of DME, helium and water as a function of time for a cyclically operated process according to the invention. The output concentration is measured by mass spectrometry. Figure 1a represents the 3^{rd} cycle from start of the reaction. The concentration is measured by mass spectrometry. On the X-axis time is plotted expressed in minutes. On the Y-axis the measured intensity is plotted. The solid black line is the helium signal (m/z = 4), the grey dashed line with long dashes is the DME signal (m/z = 45) and black dashed line with short dashes is the water signal (m/z = 18). The DME signal shows a peak at about 296.3 minutes.

Figure 1b depicts the output concentration of DME, helium and water as a function of time for a cyclically operated process according to the invention. The output concentration is measured by mass spectrometry. Figure 1b represents the 50^{th} cycle from start of the reaction. The concentration is measured by mass spectrometry. On the X-axis time is plotted expressed in minutes. On the Y-axis the measured intensity is plotted. The solid black line is the helium signal (m/z = 4), the grey dashed line with long dashes is the DME signal (m/z = 45) and the grey solid line is the water signal (m/z = 18). The water signal remains below an intensity level of 1.00E-09. The DME signal shows a peak at about 4682.3 minutes.

### Examples

### Example 1 - Oxygenate conversion

### Method

A quartz reactor with an internal diameter of 16 mm was filled with 2.5 gram of catalyst and 6.0 gram of water adsorbent resulting in a bed height of about 70 mm. During adsorption 92 Nml min⁻¹ was fed to the reactor at 250°C at 1 bara. The gas mixture contained 5 mol% DME, 5 mol% He as tracer and balance Ar. Regeneration is performed by switching the gas flow to 100 mol% Ar, and increasing the temperature to 400°C for 60 minutes. Reactor outlet gas analysis was performed continuously by a Pfeiffer Thermostar mass spectrometer.

For the materials a mixture was used which consists of adsorbent and catalyst, either SAPO or HZSM-5. The used materials are:

| | |
|---|---|
| Water adsorbent: | Molecular sieve Type 3A, ASGE |
| Catalyst 1: | SAPO-34, Zeolyst |
| Catalyst 2: | Zeolite HZSM-5, PQ Chemicals |

For the experiments a particle size of 0.212-0.425 mm is used. The adsorbent (3A) is available in the form of small pellets. By crushing and sieving the correct particle size is obtained. Both the catalysts are available as fine powder. In order to obtain the right particle size these powders are first pelletized using a manual press. Subsequently the tablets are crushed and sieved again. 6.0 g of adsorbent particles is thoroughly mixed with 2.5 gram of either SAPO-34 or HZSM-5. After mixing the 16 mm internal diameter quartz reactor is filled, resulting in a typical bed height of about 70 mm.

### Results

Mass spectrometry of the product mixtures was performed and the peaks were assignment according to Wallace, W. E. (2018). Mass spectra. *NIST Chemistry WebBook, NIST Standard Reference Database,* (69). In table 1 an overview is given of the analysed species with their corresponding m/z values. Table 2 summarizes the pre- and post breakthrough values of each m/z at specified times.

**Table 1. Mass spectrometer response and peak assignment**

| **m/z** | **Species** | | | |
|---|---|---|---|---|
| **18** | H₂O | | | |
| **31** | Methanol | | | |
| **41** | Propane | Propylene | Butane | 2-Butene |
| **43** | Propane | Butane | | |
| **45** | DME | | | |
| **55** | 2-Butene | | | |

**Table 2. Pre- and post-breakthrough product distribution (the pre- and post-breakthrough intensities normalised relative to helium, He (at t=0) = 100)**

| **Experiment 1: SAPO34 plus zeolite 3A** | | | |
|---|---|---|---|
| **m/z** | **Species** | **Pre-breakthrough (t=11.5 min)** | **Post-breakthrough plateau phase (t=100 min)** |
| 18 | Water | 0.48 | 31.39 |
| 31 | Methanol | 0.01 | 0.77 |
| 41 | Propane, Propylene, Butane, 2-Butene | 4.25 | 6.52 |
| 43 | Propane, Butane | 0.51 | 0.56 |
| 45 | DME | 0.01 | 4.78 |
| 55 | 2-Butene | 1.35 | 0.77 |

| **Experiment 2: H-ZSM-5 plus zeolite 3A** | | | |
|---|---|---|---|
| **m/z** | **Species** | **Pre-breakthrough (t=6.5 min)** | **Post-breakthrough plateau phase (t=65 min)** |
| 18 | Water | 0.91 | 23.13 |
| 31 | Methanol | 0.04 | 9.29 |
| 41 | Propane, Propylene, Butane, 2-Butene | 7.49 | 5.23 |
| 43 | Propane, Butane | 6.68 | 3.48 |
| 45 | DME | 0.11 | 21.19 |
| 55 | 2-Butene | 1.85 | 0.81 |

### Discussion and conclusion

Both experiments concern DME dehydration experiments over SAPO and HZSM5 catalysts, much like these are conventionally performed. The reaction temperature is relatively low. The difference is in the presence of the water adsorbent which affects the reactor performance and creates a transient response.

Essentially, the experiments can be divided in pre- and post-breakthrough periods. The pre-breakthrough period starts at t=0, and ends when the water signal starts to increase. This increase signals that water starts to pass the reactor and 3A water adsorbent: the adsorbent becomes saturated. The post-breakthrough period comprises two phases, first the water concentration in the reaction product increases logarithmically, until a plateau phase is reached. For experiment 1 the pre-breakthrough period ends at about 28 minutes and the plateau phase is reached at about 65 minutes, for experiment 2 the pre-breakthrough period ends at about 4 minutes and the plateau phase is reached at about 33 minutes.

Before water breakthrough, the concept of adsorption enhancement applies: the adsorption removes water from the gas phase in the reactor, causing a shift or enhancement in DME conversion. As table 2 demonstrates both for experiment 1 and 2 the amount of the oxygenates DME and methanol leaving the reactor is very low.

The plateau phase represent a 'conventional' response, as the effect of the adsorbent is effectively cancelled. Whereas mass spectrometer signals are not quantitative, they can be compared in relation to each other. For experiment 1 and 2, the response clearly shows a significantly enhanced DME (and methanol) conversion in pre-breakthrough compared to post-breakthrough. In the pre-breakthrough period, experiment 1 shows a high response in e.g. m/z values of 41 and 55 indicative of hydrocarbons (olefins and/or paraffins) formed. Experiment 2 shows this effect eve more pronounced, where pre-breakthrough m/z values of 41, 43, and 55 are higher than their respective post-breakthrough responses. In all cases, after water breakthrough, the DME response strongly increases, which indicates the DME conversion drops significantly. The low conversion for the conventional process without adsorption is not unexpected, since the temperature of 250 °C is rather low for DME (and methanol) dehydration experiments.

In conclusion, experiment 1 and experiment 2 show the effect of sorption-enhancement. Pre-breakthrough a near-complete conversion of DME can be achieved, and hydrocarbon products are formed. Post-breakthrough conditions show incomplete conversion as well as a somewhat modified product spectrum. The sorption enhancement therefore increases conversion considerably and enables a lower reaction temperature.

### Example 2 - Absence of water adsorption by SAPO34 catalyst

The SAPO34 catalyst of example 1 was tested for water adsorption. 0.5 gram of SAPO34 catalyst (sieve fraction of 212-425 µm) was placed in a reactor with a diameter of 6mm and the temperature was brought to 250°C. The pressure in the reactor was 1 bara. A gas stream (92 Nml min⁻¹) comprising 10 mole% water was provided to the reactor and the gas flow coming from the reactor was analysed as in example 1. Breakthrough of the water front was instantaneous and within less than a minute a constant water concentration in the outgoing stream was reached, indicating no water adsorption took place. A steady state flow was kept for about 22 minutes and after that the temperature was increased to 400°C and a gas flow was provided that did not contain water. The subsequent drop in water concentration in the outgoing stream was instantaneous, no water peak which is typical for a water adsorbing material was observed again indicating the SAPO34 catalyst does not adsorb water.

### Example 3 - Absence of catalyst deactivation by coke formation

### Method

For example 3 the water adsorber combination of experiment 1 (zeolite HZSM-5, PQ Chemicals and molecular sieve Type 3A, ASGE; both in sieve fraction of 212-425 µm) was used. A quartz reactor with an internal diameter of 10 mm was filled with 0.5 gram of catalyst and 2.5 gram of water adsorbent resulting in a bed height of about 58 mm. However instead of monitoring one cycle, the reactor was operated alternating in reaction mode and sorbent regeneration mode. The reaction mode was as described in example 1, with a temperature of 250°C and a pressure of 1 bara. The sorbent regeneration mode was at 400°C and 1 bara. As for example 1 during adsorption 92 Nml min⁻¹ was fed to the reactor. The gas mixture contained 5 mol% DME, 5 mol% He as tracer and balance Ar. The purge gas was Ar. Reaction time was 15 minutes and regeneration time 45 minutes. Reactor outlet gas analysis was performed continuously by a Pfeiffer Thermostar mass spectrometer. At the start of the reaction mode, following the sorbent regeneration mode, the reactor is fed with DME and the helium inlet concentration is raised (step change). At the outlet of the reactor, the concentrations of reaction products and unconverted feed gases are measure in time. For clarity, only the outlet concentrations of helium, DME, and water are plotted.

### Results and conclusion

The aim of this experiment was to investigate the effect of the method of the invention on catalyst deactivation. In figure 1a the outlet concentration of DME (m/z 45), He (m/z 4) and water (m/z 18) is plotted as a function of time for the 3^{rd} cycle, in figure 1b for the 50^{th} cycle.

The performance of the system is assessed in terms of the DME conversion. As in Example 1 and Example 2, the breakthrough of the increased helium concentration is followed by a period in which all DME is converted, until DME breaks through as well, followed by water breakthrough. Both the local peak of DME that can be observed just after DME breakthrough, and the difference in breakthrough time between DME and water, are not further investigated but tentatively attributed to a dynamic 'roll up' effect (as discussed for example in Yang, R. T. (1997). Gas separation by adsorption processes (Vol. 1). World Scientific.).

For cycle 3 the He signal is steady and increases at t=295.7 minutes indicating the end of the regeneration mode and the start of the reaction mode. Breakthrough of DME is at 296.2 minutes; 0.42 minutes after start of the reaction mode. For cycle 50 the He signal is steady and increases at t=4681.8 minutes indicating the end of the regeneration mode and the start of the reaction mode. Breakthrough of DME is at 4682.2 minutes; 0.43 minutes after start of the reaction mode.

Essentially, the DME conversion, as derived from the difference in breakthrough time between the helium step change and the DME signal, surprisingly remains unchanged between cycle 3 and cycle 50. Consequently, during the more than 70 hours that the cycles were repeated, no measurable degradation of DME conversion can be observed, from which it can be concluded that the catalyst activity remains constant.

## Claims

1. Use of a sorbent material capable of adsorbing water for converting oxygenates to hydrocarbons in the presence of a catalyst for conversion of oxygenates to hydrocarbons wherein the catalyst and sorbent material are not the same, preferably wherein the use involves one or more of the following:
- the conversion is increased compared to when the sorbent material is not present,
- the lifetime of the catalyst is elongated compared to when the sorbent material is not present,
- the deactivation of the catalyst is reduced compared to when the sorbent material is not present,
- the reaction rate is increased compared to when the sorbent material is not present, and
- the need for catalyst decoking is reduced preferably prevented compared to when the sorbent material is not present.

2. A process for the conversion of oxygenates to hydrocarbons comprising:
a) in a reactor contacting a catalyst with the oxygenate in the presence of a sorbent material capable of adsorbing water to form the hydrocarbons; and subsequently
b) regenerating the sorbent material in the same reactor by contacting the sorbent material with a purge gas;
wherein the catalyst and sorbent material are not the same.

3. The process according to claim 2, wherein the process is operated cyclically wherein step b) is followed by step a).

4. The process according to claim 2 or 3, wherein the temperature in step a) is between 200°C and 500°C, preferably between 200°C and 400°C, even more preferably between 200°C and 350°C, most preferably between 225°C and 275°C.

5. The process according to any one of claims 2-4, wherein the pressure in step a) is between 1.0 and 30 bara, preferably between 1.0 and 20 bara.

6. The process according to any one of claims 2-5, wherein the temperature in step b) is at least 100°C higher than the temperature of step a) and/or wherein the pressure of step b) is at least 0.9 bar lower than the pressure of step a), preferably wherein the temperature in step b) is at least 150°C higher than the temperature of step a) and/or wherein the pressure of step b) is at least 3.0 bar lower than the pressure of step a).

7. The process according to any one of claims 2-6, having two or more reactors comprising the catalyst and the sorbent material capable of adsorbing water, preferably wherein at least one reactor of the two or more reactors is performing step a).

8. The process according to any one of claims 2-7, wherein the catalyst is selected from the list consisting of H-beta, ZSM-5, ZSM-22, MCM-22, mordenite, UZM-9, SAPO (such as SAPO-17,-18, - 34, -35, -44, -56) and AIPO, preferably the catalyst is ZSM-5 or SAPO-34.

9. The process according to any one of claims 2-8, wherein the sorbent material capable of adsorbing water is a molecular sieve having a pore diameter in the range of 2-5 Å, more preferably a zeolite having a pore diameter in the range of 2-5 Å, even more preferably an LTA zeolite having a pore diameter in the range of 2-5 Å.

10. The process according to any one of claims 2-9, wherein the weight ratio of catalyst to sorbent material capable of adsorbing water is between 1.0:10.0 and 10.0:1.0, preferably between 1.0:10.0 and 5.0:1.0.

11. The process according to any one of claims 2-10, wherein the sorbent material capable of adsorbing water has a water adsorption capacity of at least 0.04 gram water per gram sorbent material at the temperature and pressure in step a), preferably at least 0.06 gram water per gram sorbent material at the temperature and pressure in step a), more preferably at least 0.08 gram water per gram sorbent material at the temperature and pressure in step a), most preferably at least 0.10 gram water per gram sorbent material at the temperature and pressure in step a).

12. The process according to any one of claims 2-11, wherein the catalyst has a water adsorption capacity of less than 0.10 gram water per gram catalyst at the temperature and pressure in step a), preferably less than 0.04 gram water per gram catalyst at the temperature and pressure in step a).

13. The process according to any one of claims 2-12, wherein the oxygenate is one or more of methanol (MeOH), ethanol (EtOH), isopropyl alcohol (IPA), n-butanol (BuOH), gasoline grade tert-butanol (GTBA), methyl tert-butyl ether (MTBE), tert-amyl methyl ether (TAME), tert-hexyl methyl ether (THEME), ethyl tert-butyl ether (ETBE), tert-amyl ethyl ether (TAEE), dimethylether (DME) or diisopropyl ether (DIPE), preferably the oxygenate is methanol and/or dimethylether, more preferably the oxygenate is methanol.

14. The process according to any one of claims 2-13, wherein the hydrocarbon is selected from the group consisting of olefins, alkanes, aromatics and any combination thereof, wherein preferably the olefin is one or more of ethylene, propylene, 1-butene, 2-butene, and isobutylene, the alkane is one or more of alkanes comprising 2 to 18 carbon atoms and the aromatic is one or more of benzene, toluene and xylene.

15. A fluidized or fixed bed reactor for conversion of oxygenates to hydrocarbons comprising a catalyst for conversion of oxygenates to hydrocarbons and a sorbent material capable of adsorbing water, wherein the catalyst and the sorbent material are not the same.
